# EUROPÄISCHE PATENTSCHRIFT

(11) **EP 0 354 419 B1**
(45) Veröffentlichungstag und Bekanntmachung des Hinweises auf die Patenterteilung: **27.01.1993**
(21) Anmeldenummer: 89113927.1
(22) Anmeldetag: 28.07.1989
(51) Int. Cl.: C07C 59/135, C07C 51/58, C07C 53/50

(54) **Verfahren zur Herstellung von fluorierten Carbonsäurefluoriden**
Method for the production of fluorinated carboxylic acid fluorides
Procédé pour la fabrication de fluorures d'acides carboxyliques fluorés

(30) Priorität: 06.08.1988 DE 3826807
(43) Veröffentlichungstag der Anmeldung: 14.02.1990
(73) Patentinhaber: HOECHST AKTIENGESELLSCHAFT, 65926 Frankfurt am Main (DE)
(72) Erfinder: Schwertfeger, Werner, Dr., D-6306 Langgöns (DE); Hintzer, Klaus, Dr., D-8269 Burgkirchen (DE); Blickle, Peter, Dr., D-6233 Kelkheim (Taunus) (DE)

(56) Entgegenhaltungen:
- US-A- 3 442 942
- US-A- 3 451 908

## Beschreibung

Die Erfindung bezieht sich auf die Herstellung von fluorierten Carbonsäurefluoriden aus fluorierten Vinylethern.

Fluorierte Carbonsäuren und deren Derivate finden in der Technik vielseitige Verwendungsmöglichkeiten.
Die Salze langkettiger perfluorierter Carbonsäuren werden als Emulgatoren bei der Herstellung von Polytetrafluorethylen eingesetzt, während fluorierte Carbonsäuren in Form ihrer Säurefluoride für die Synthese von fluorierten Vinylethern benötigt werden. Fluorierte Carbonsäuren werden in die entsprechenden Kolbe-Produkte umgewandelt, die als Inerflüssigkeiten bzw. als Lösemittel für fluorierte Harze dienen, und perfluorierte Carbonsäurefluoride werden durch Belichtung in perfluorierte Inertflüssigkeiten überführt.

Durch Reaktionen von fluorierten Säurefluoriden mit fluorierten Epoxiden, beispielsweise Hexafluorpropenoxid (HFPO) oder Tetrafluorethylenoxid (TFEO), entstehen fluorierte Carbonsäurefluoride, die Ethergruppen enthalten. Beim Einsatz von HFPO sind die Produkte immer verzweigt, während TFEO eine schwer herstellbare und handhabbare Substanz ist (vgl. H. Millauer et al., Angew. Chem., Int. Ed. Engl. 24 (1985) 161;
P. Tarrant et al., Fluor.Chem.Rev., 5 (1971) 77).

Perfluorierte Olefine liefern bekanntermaßen in Gegenwart von Sauerstoff und einer Lewis-Säure wie SbF₅ keine sauerstoffhaltigen Verbindungen. Es tritt allerdings Isomerisierung ein (vgl. CA 91 38844v; CA 94 102806a).

Es bestand nun die Aufgabe, ein Verfahren zur Herstellung von fluorierten Carbonsäuren bzw. deren Derivaten zu finden, das von technisch genutzten Ausgangsmaterialien ausgeht und fluorierte Carbonsäurefluoride unterschiedlicher Struktur durch Anwendung nur eines Reaktionstyps zu liefern imstande ist.

Es wurde gefunden, daß fluorierte Vinylether in Gegenwart eines Sauerstoff enthaltenden Gases und mit Hilfe einer katalytischen Menge einer Lewis-Säure fluorierte Carbonsäurefluoride bilden.

Die Erfindung betrifft somit das Verfahren gemäß den Ansprüchen.

Fluorierte Carbonsäurefluoride mit der Formel I 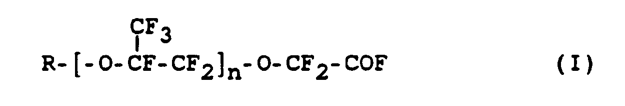
worin R ein verzweigter oder unverzweigter perfluorierter Rest mit 1-10, vorzugsweise 1-7 Kohlenstoffatomen bedeutet, in dem ein oder mehrere Fluoratome durch andere Halogenatome oder ein Wasserstoffatom substituiert sein können, und n eine ganze Zahl von null bis 10, vorzugsweise von eins bis 5, insbesondere 1 oder 2 ist, werden aus Vinylethern der Formel II 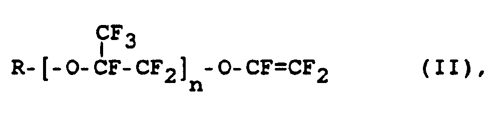
mit R und n gemäß Formel I in Gegenwart einer katalytischen Menge einer Lewis-Säure, beispielsweise SbF₅, TiCl₄, HF oder BF₃ oder Mischungen von Lewis-Säuren, durch Umsetzung mit einem Sauerstoff enthaltenen Gas, vorzugsweise O₂ oder Luft, hergestellt.

Die Umsetzung des Vinylethers der Formel II erfolgt in einem Gefäß aus Glas, Metall oder Kunststoff.

Als Glasgefäße kommen beispielsweise Kolonnen, Reaktionsrohre sowie Kolben zur Verwendung. Die Kolben sind mit einem Magnetrührer, Thermometer, Kühler und Gaseinleitungsrohr versehen.

In das Reaktionsgefäß wird der Vinylether gegeben und das Sauerstoff enthaltende Gas eingeleitet. Der Fortgang der Reaktion wird durch Entnahme von Proben und Untersuchungen dieser Proben durch Gaschromatographie oder Infrarotspektralanalyse überwacht. Der Katalysator wird zu Beginn des Einleitens zugesetzt, kann aber nachdosiert werden, wenn der Umsatz des Vinylethers noch nicht vollständig oder die Reaktionsgeschwindigkeit nicht mehr ausreichend hoch ist. Es ist vorteilhaft, während der gesamten Reaktion zu rühren. Die Reaktion wird in einem Temperaturbereich von -50 bis +200°C, vorzugsweise von 0 und 100°C und bei Normal-, Unter- oder Überdruck, vorzugsweise bei Normal- oder Überdruck, durchgeführt.

Der Katalysator wird in Mengen von 0,01 bis 20 mol%, bezogen auf den Vinylether, eingesetzt.

Bei der Verwendung von Autoklaven wird die Reaktion bei einem Überdruck eines Sauerstoff enthaltenden Gases, vorzugsweise Sauerstoff und Luft, insbesondere Sauerstoff, durchgeführt.

Die erfindungsgemäße Reaktion liefert Verbindungen der Formel I, die bisher nur durch Reaktion des schwierig herstellbaren und handhabbaren Tetrafluorethylenoxid (TFEO) 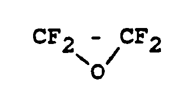
mit fluorierten Caronsäuren bzw. Ketonen herstellbar waren.

Nach der Reaktion können die Produkte durch Folgereaktionen, beispielsweise Verseifung, Veresterung oder Aminolyse, vorzugsweise Veresterung, in ihre entsprechenden Carbonsäuren oder deren Derivate, beispielsweise Ester und Amide, vorzugsweise Ester, überführt werden.

### Beispiele

### Beispiel 1

In einem Glaskolben, der mit einem Magnetrührer, Thermometer, Kälte-Kühler und Gaseinleitungsrohr ausgestattet war, wurden 133g (0,5 mol) CF₃-CF₂-CF₂-O-CF=CF₂ (Perfluorpropylvinylether =PPVE) vorgelegt. Danach wurde Sauerstoff bei Zimmertemperatur eingeleitet und ca. 100 mg SbF₅ hinzugesetzt. Sofort trat eine exotherme Reaktion ein und das Gemisch begann zu sieden. Nachdem die Reaktion abgeklungen war, wurde der Ansatz über Nacht bei Zimmertemperatur stehen gelassen. Eine Probe des Ansatzes wurde mit Methanol verestert, dabei wurde das bei der Reaktion entstandene CF₃-CF₂-CF₂-O-CF₂-COF zu CF₃-CF₂-CF₂O-CF₂-COOCH₃ verestert, und anschließend ein GC (WLD) aufgenommen. Es zeigte die Zusammensetzung:
- 83 %: PPVE
- 3 %: CF₃-CF₂-COOCH₃
- 13 %: CF₃-CF₂-CF₂-O-CF₂-COOCH₃.

Anschließend wurde der gesamte Ansatz mit Methanol verestert und destilliert, dabei hatte CF₃-CF₂-CF₂-O-CF₂-COOCH₃ einen Siederpunkt von 102-104°C. Die Ausbeute des Esters betrug 9 g.

### Beispiel 2

In der Apparatur gemäß Beispiel 1 wurden bei Zimmertemperatur 108 g (0,2 mol) Perfluorpropoxypropylvinylether 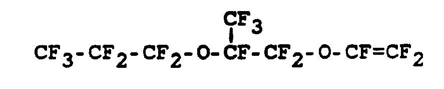
(PPVE-2) vorgelegt und Sauerstoff eingeleitet. Anschließend wurden ca. 100 mg SbF₅ hinzugegeben. Nach der Reaktion sind weitere 100 mg SbF₅ hinzugesetzt worden, wodurch die Temperatur innerhalb des Kolbens auf 60°C anstieg. Nach Beendigung der Reaktion wurden alle flüchtigen Bestandteile bei 10-15 mbar in eine Kühlfalle, die eine Temperatur von -78°C hatte, abdestilliert.

Die Kühlfalle enthielt 103 g einer farblosen Flüssigkeit. Eine Probe des Ansatzes wurde mit Methanol verestert und anschließende ein GC (WLD) aufgenommen. Es zeigte die Zusammensetzung:

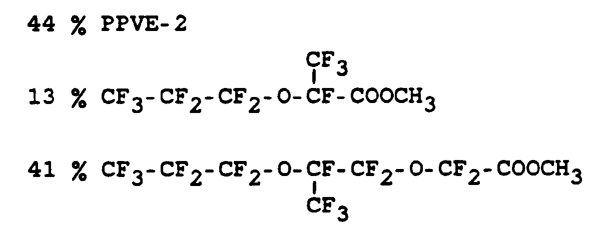

Anschließend wurde der gesamte Ansatz mit Methanol verestert und destilliert, dabei hatte 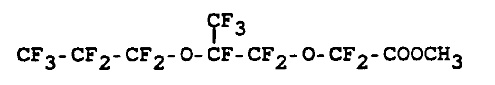
einen Siedepunkt von 91-92°C bei 133 mbar.

### Beispiel 3

In einem 100 ml Glaskolben mit Magnetrührer, Thermometer, Kälte-Kühler und kurzem Gaseinleitungsrohr legte man 100 g 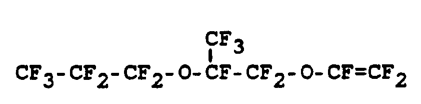
(PPVE-2) vor. Nach Zugabe von 1,5 g SbF₅ leitete man Luft ein. Es trat eine exotherme Reaktion auf. Man ersetzte die eingeleitete Luft durch reinen Sauerstoff. Die exotherme Reaktion wurde deutlich stärker. Die maximal erreichte Innentemperatur betrug 68,4°C. Im abgehenden Gas wurde mit Hilfe der IR-Spektroskopie COF₂ nachgewiesen. Nach Abklingen der exothermen Reaktion setzte man 1 g SbF₅ zu, wonach erneut eine exotherme Reaktion eintrat. Dieser Vorgang wurde noch einmal wiederholt. Im Kolbeninhalt war danach IR-spektroskopisch kein PPVE-2 mehr nachweisbar. Man zog alles Flüchtige bei 10 mbar aus dem Reaktionskolben in eine bei -78°C gehaltene Kältefalle. Der Falleninhalt (87 g) wurde mit Methanol verestert, mit Wasser gewaschen und über Natriumsulfat getrocknet. Das Gaschromatogramm (WLD) des veresterten Gemisches zeigte neben 4 % PPVE-2

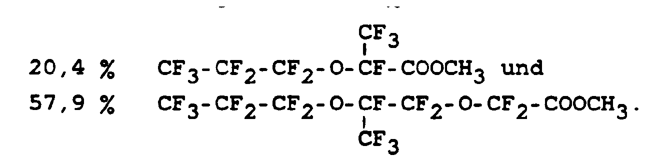

Die Destillation lieferte

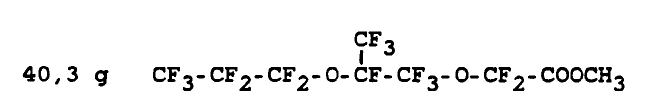

mit einem Kp von 78°C/67 mbar.

### Beispiel 4

In der Apparatur gemäß Beispiel 1 wurden bei Zimmertemperatur 108 g (0,25 mol) PPVE-2 vorgelegt und Sauerstoff eingeleitet. Anschließend wurde ca. 1 g TiCl₄ hinzugegeben.

Während der exothermen Reaktion wurde Sauerstoff aufgenommen. Eine Probe des Ansatzes wurde mit Methanol verestert und anschließend ein GC (WLD) aufgenommen. Es zeigte die Zusammensetzung:

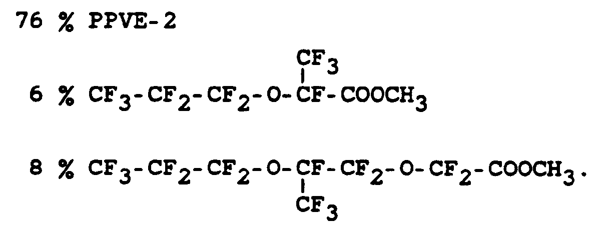

Nach der Reaktion wurde der Ansatz unter Vakuum in eine Kühlfalle destilliert und vom Inhalt (78 g) ein ¹⁹F-NMR-Spektrum aufgenommen worden, das das Ergebnis der GC-Untersuchung bestätigte. Außerdem zeigte das Spektrum das Signal für COF₂.

### Beispiel 5

In ein PE-Gefäß wurden 108 g (0,25 mol) PPVE-2 und 1 g wasserfreie HF gegeben. Nach Durchschütteln wurden 9 g der unteren Phase in einen 200 cm³ Autoklaven aus rostfreiem Stahl gefüllt und unter einem Sauerstoff-Druck von 10 bar 20 Stunden bei 50°C erhitzt.

Nach dem Abkühlen wurde eine Probe des flüssigen Autoklaveninhalts verestert und anschließend ein GC(WLD) aufgenommen. Es zeigte die Zusammensetzung:

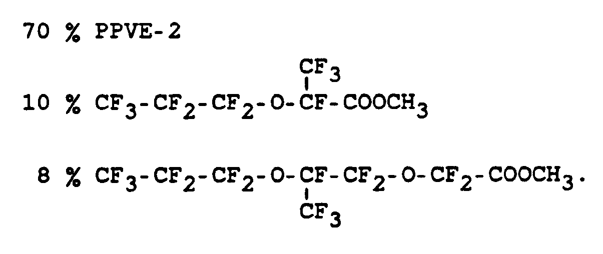

### Beispiel 6

In der Apparatur gemäß Beispiel 1 wurden bei Zimmertemperatur 104,4 g (0,3 mol) H-(CF₂)₅-O-CF=CF₂ vorgelegt und Sauerstoff eingeleitet. Anschließend wurden zweimal je 100 mg SbF₅ hinzugegeben, wobei eine exotherme Reaktion einsetzte.

Der Ansatz wurde mit Methanol verestert. Dabei wurde das bei der Reaktion entstandene H(CF₂)₄-O-CF₂-COF zu H-(CF₂)₄-O-CF₂-COOCH₃ verestert. Danach wurde der Ansatz mit Wasser gewaschen und über Na₂SO₄ getrocknet. Anschließend wurde ein GC(WLD) aufgenommen. Es zeigte die Zusammensetzung:
- 60 %: H-(CF₂)₅-O-CF=CF₂
- 10 %: H-(CF₂)₄-COOCH₃
- 27 %: H-(CF₂)₅-O-CF₂-COOCH₃.

Bei der Destillation hatte der Ester H-(CF₂)₅-O-CF₂-COOCH₃ einen Siedepunkt von 163-166°C.

### Beispiel 7

In der Apparatur gemäß Beispiel 1 wurden 55,4 g (0,2 mol) Br-CF₂-CF₂-O-CF=CF₂ unter Luft mit ca. 100 mg SbF₅ versetzt. Nach der schwach exothermen Reaktion leitete man einen schwachen Luftstrom in die Apparatur.

Nach einer Reaktionszeit von 20 Stunden bei Zimmertemperatur wurde der Ansatz mit Methanol verestert. Nach Aufarbeitung mit Wasser und Trocknen wurde ein GC (WLD) aufgenommen. Es zeigte die Zusammensetzung:
- 48 %: Br-CF₂-CF₂-O-CF=CF₂
- 10 %: Br-CF₂-COOCH₃
- 39 %: Br-CF₂-CF₂-O-CF₂-COOCH₃

Bei der Destillation hatte der Ester Br-CF₂-CF₂-O-CF₂-COOCH₃ einen Siedepunkt von 126-131°C.

### Beispiel 8

In der Apparatur des Beispiels 1 versetzte man 50 g (0,18 mol) Br-CF₂-CF₂-O-CF=CF₂ mit 1 g SbF₅ und leitete Sauerstoff ein. Nach dem Abklingen der exothermen Reaktion wurde erneut 1 g SbF₅ zugegeben. Nachdem die wieder auftretende exotherme Reaktion beendet war, zog man alles Flüchtige bei 67 mbar in eine mit Trockeneis gekühlte Kältefalle. Bei Raumtemperatur enthielt die Falle 40 g farblose Flüssigkeit. Eine Probe wurde mit Methanol verestert und mit Hilfe der Gaschromatographie (WLD) untersucht. Die Probe enthielt
- 3,1 %: Br-CF₂-CF₂-O-CF=CF₂
- 26,5 %: Br-CF₂-COOCH₃
- 63,8 %: Br-CF₂-CF₂-O-CF₂-COOCH₃

### Vergleichsbeispiel

In der Apparatur gemäß Beispiel 1 wurden bei Zimmertemperatur 50 g eines Gemisches isomerer Perfluorheptene, das sich nach dem Gaschromatogramm und dem ¹⁹F-NMR-Spektrum aus CF₃-(CF₂)₄-CF=CF₂ sowie CF₃-(CF₂)₃-CF=CF-CF₃ (cis und trans) im Molverhältnis 10:6,2 zusammensetzte, vorgelegt und Sauerstoff eingeleitet. Anschließend wurden ca. 100 mg SbF₅ hinzugegeben.

Es setzte sofort eine exotherme Reaktion ein. Nach dem Abklingen der Reaktion wurde das Gemisch mit Hilfe von ¹⁹F-NMR-Spektroskopie untersucht. Es waren keine Produkte nachweisbar, die auf eine Reaktion mit Sauerstoff hinwiesen. Statt dessen waren ausschließlich die isomeren Olefine CF₃-(CF₂)₃-CF=CF-CF₃ (cis und trans) vorhanden.

## Patentansprüche

1. Verfahren zur Herstellung von fluorierten Carbonsäurefluoriden der Formel I 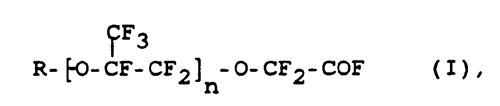 worin R ein verzweigter oder unverzweigter perfluorierter Rest mit 1-10 Koklenstoffatomen bedeutet, in dem ein oder mehrere Fluoratome durch andere Halogenatome oder ein Wasserstoffatom substituiert sein können, und n eine ganze Zahl von null bis 10 ist, dadurch gekennzeichnet, daß ein Vinylether der Formel II 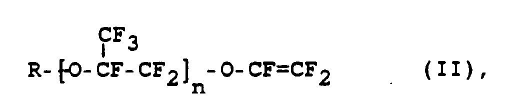 mit R und n gemäß Formel I, in Gegenwart einer katalytischen Menge einer Lewis-Säure mit einem Sauerstoff enthaltenden Gas umgesetzt wird.

2. Verfahren nach Anspruch 1, dadurch gekennzeichnet, daß die Reaktion in einem Temperaturbereich von -50 bis +200°C durchgeführt wird.

3. Verfahren nach Anspruch 1, dadurch gekennzeichnet, daß das Sauerstoff enthaltenden Gas Sauerstoff oder Luft ist.

4. Verfahren nach Anspruch 1, dadurch gekennzeichnet, daß die Lewis-Säure in einer Menge von 0,01 bis 20 mol% eingesetzt wird.

5. Verfahren nach Anspruch 1, dadurch gekennzeichnet, daß die Lewis-Säure SbF₅, TiCl₄, HF oder BF₃ ist.

## Claims

1. A process for the preparation of a fluorinated carboxylic acid fluoride of the formula I 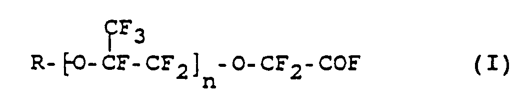 in which R denotes a branched or non-branched perfluorinated radical having 1-10 carbon atoms, in which one or more fluorine atoms can be replaced by other halogen atoms or a hydrogen atom, and n is an integer from zero to 10, which comprises reacting a vinyl ether of the formula II 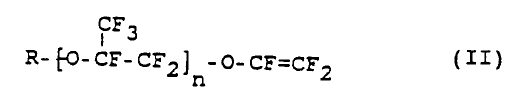 with R and n according to formula I, with an oxygen-containing gas in the presence of a catalytic amount of a Lewis acid.

2. The process as claimed in claim 1, wherein the reaction is carried out in a temperature range from -50 to +200 °C.

3. The process as claimed in claim 1, wherein the oxygen-containing gas is oxygen or air.

4. The process as claimed in claim 1, wherein the Lewis acid is employed in an amount of 0. 01 to 20 mol %.

5. The process as claimed in claim 1, wherein the Lewis acid is SbF₅, TiCl₄, HF or BF₃.

## Revendications

1. Procédé pour préparer des fluorures d'acides carboxyliques fluorés de formule I : 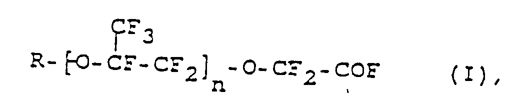 (dans laquelle R représente un reste perfluoré, ramifié ou non ramifié, comportant 1 à 10 dans lequel un ou plusieurs atomes de fluor peuvent être remplacés par d'autres atomes d'halogènes ou par un atome d'hydrogène ; et n est un nombre entier valant de 0 à 10), procédé caractérisé en ce qu'on fait réagir un ester vinylique de formule II : 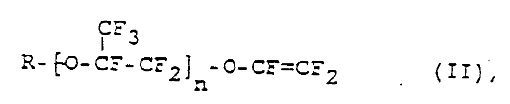 (dans laquelle R et n ont le sens indiqué pour la revendication 1) en présence d'une quantité catalytique d'un acide de Lewis, avec un gaz contenant de l'oxygène.

2. Procédé selon la revendication 1, caractérisé en ce qu'on conduit la réaction dans un intervalle de la température allant de -50 à +200°C.

3. Procédé selon la revendication 1, caractérisé en ce que le gaz contenant de l'oxygène est de l'oxygène ou de l'air.

4. Procédé selon la revendication 1, caractérisé en ce qu'on utilise l'acide de Lewis en une quantité de 0,01 à 20 mole%.

5. Procédé selon la revendication 1, caractérisé en ce que l'acide de Lewis est SbF₅, TiCl₄, HF ou BF₃.
